# EUROPEAN PATENT SPECIFICATION

(11) **EP 0 477 994 B1**
(45) Date of publication and mention of the grant of the patent: **13.06.2001**
(21) Application number: 91117193.2
(22) Date of filing: 17.03.1986
(51) Int. Cl.: C12N 15/00, C12N 1/21, C12N 5/10, C12N 15/84

(54) **Parasite-derived resistance**
Von Parasit gewonnener Widerstand
Résistance dérivée de parasite

(30) Priority: 21.03.1985 US 714263
(43) Date of publication of application: 01.04.1992
(62) Divisional of application: 86902165.9
(73) Proprietor: Johnston, Stephen, Ph.D., Dallas, TX 75235-8573 (US); CORNELL RESEARCH FOUNDATION, INC., Ithaca, N.Y. 14850 (US)
(72) Inventor: Johnston, Stephen A., Dallas, Texas 75229 (US); Sanford, John C., Geneva, New York, 14456 (US)
(74) Representative: Giambrocono, Alfonso, Dr. Ing.

(56) References cited:
- EP-A- 0 140 308
- EP-A- 0 194 809
- WO-A-83/01451
- WO-A-85/04898
- GB-A- 2 148 302
- PROC. NATL. ACAD. SCI. USA, vol. 75, no. 1, January 1978, pages 280-284; P.C. ZAMECNIK et al.: "Inhibition of Rous sarcoma virus replication and cell transformation by a specific oligodeoxynucleotide"
- PROC. NATL. ACAD. SCI. USA, vol. 75, no. 1, January 1978, pages 285-288; M.L. STEPHENSON et al.: "Inhibition of Rous sarcoma viral RNA translation by a specific oligodeoxyribonucleotide"
- PLANT MICROBE INTERACTIONS, 1984, pages 420-429, Chapter 17, Mcmillan Inc.; P. PALUKAITIS et al.: "A model to explain the "cross-protection" phenomenon shown by plant viruses and viroids"
- J. THEOR. BIOL., vol. 113, no. 2, 21st March 1985, pages 395-405, Academic Press Inc., Ltd, London, GB; J.C. SANFORD et al.: "The concept of Parasite-derived resistance - deriving resistance genes from the parasite's own genome"

## Description

### Technical Field

The present invention relates to methods of conferring resistance to parasites, such as viruses, bacteria, and higher parasites, to hosts of the parasite. More particular, this invention relates to parasite resistance obtained by genetic engineering of the host organism.

### Background Art

A potentially important application of genetic engineering technology is in the area of producing resistance to parasites. The proposals in the prior art that have been systematic and broadly applicable have centered on finding a gene conferring resistance within a strain of the host species or within a related species and transforming the gene into the genome of a susceptible host. This approach may prove effective hut has several distinct disadvantages. Resistant forms of the host may not exist or nay be very difficult to find for each new race of parasite which arises. Such resistance nay be polygenic, making the cloning and transfer of the resistance genes difficult. Where resistance is encoded by a gene, there are commonly already strains of the parasite that have evolved virulence genes for overcoming such host-derived resistances in a gene-for-gene fashion (Flor 1971). Finally, the problem of identifying and isolating the resistance gene from within the large genome of the host will generally remain very difficult. An alternative strategy that addresses these problems is therefore needed.

There have also been proposals for and some work on using genes from organisms unrelated to either host or parasite, which serendipitously have gene products detrimental to a specific parasite. The gene coding for the endotoxin of Bacillus thuringiensis (which is toxic to lepidopterus insects) would be an example of this (Held et al., 1982). While this type of approach may prove useful in some specific cases, it clearly represents an opportunistic approach to the problem, as opposed to a systematic methodology that can be applied very broadly.

There already exist some examples of genes, gene derivatives, or gene products of a parasite that can produce a negative interaction with itself or a related genotype. Studies into the susceptibility of plants to infection by viruses have demonstrated that closely related plant viruses or different strains of the same virus will cross-protect a host organism (Hamilton, 1980). In other words, a plant infected by a first virus is often not subject to infection by a second strain of that virus or by a related virus. A similar phenomenon has been observed in animal viruses and has been termed intrinsic interference (Marcus and Carrier, 1967). From the point of view of parasite resistance of the type discussed herein, tile key proteins involved in the intrinsic interference phenomenon are the viral replicase proteins (Marcus and Zuckerbraun, 1970). These same authors proposed that the replicase proteins of the primary infecting virus prevent the replication of the second virus by binding to its replicase attachment sites (Marcus and Zuckerbraun, 1969). A similar proposal has been put forth to explain cross-protection in plants (Gibbs, 1969). In a similar manner, experimenters working with an E. coli infected with bacteriaphage 434 have found that infected bacteria are immune to other phages (Lauer et al, 1981; Flashman, 1973; Roberts et al, 1979). Other workers have noticed that endogenous as well as experimentally introduced complementary oligonucleotides can interact with mRNA in a potentially detrimental manner. Simons and coworkers (1983) have suggested that hybridization of a small anti-sense transcript to E. coli Tn10 mRNA contributes to the regulation of transposition of that element. Stephenson and Zamecnik (1978) and Zamecnik and Stephenson (1978) have shown that synthetic oligodeoxynucleotides, complementary to Rous sarcoma virus terminal repeats, diminish normal viral infection and can inhibit viral RNA translation in vitro. However, these discoveries were not applied to the production of host resistance to a parasite.

Despite this fragmentary knowledge in the prior art, there still remains a need for a fully developed technique for producing resistance to parasites that is not based on the traditional methods of using a resistance gene from an immune strain of a host.

### Disclosure of the Invention

According, it is an object of this invention to provide a method of conferring resistance to a parasite to a host of the parasite which does not rely on the necessity of identifying and isolating a resistance gene from an immune strain of the host.

This and other objects of the invention as will hereinafter become more readily apparent have been accomplished by providing a method for conferring resistance in a host to a parasite thereof, which comprises ;
(a) isolating a gene fragment from said parasite, and
(b) inserting in a manner to provide a heritable characteristic said gene fragment or a DNA or RNA segment substantially homologous to at least a part of said gene fragment or to a DNA or RNA sequence functionally equivalent to said gene fragment into said host,
wherein said gene fragment, or said DNA or RNA segment in said host is transcribed in an anti-sense direction, and the transcription product of said gene fragment or said DNA or RNA segment interferes with the parasite functions and reproduction.

This application is a divisional of our copending application No. 86902165.9 - 2105 filed November 10, 1986.

### Best Mode for Carrying Cut the Invention

The concept of parasite-derived resistance is that host resistance to a particular parasite can effectively be engineered by introducing a gene, gene fragment, or modified gene or gene fragment of the pathogen into the host. This approach is based upon the fact that in any parasite-host interaction, there are certain parasite-encoded cellular functions (activities) that are essential to the parasite but not to the host. An essential function is one which must operate if the parasite is to survive or reproduce. These functions represent the Achilles heel of the parasite. If one of these functions is disrupted, the parasitic process will he stopped. "Disruption" refers to any change that diminishes the survival, reproduction, or infectivity of the parasite. Such essential functions, which are under the control of the parasite's genes, can be disrupted by the presence of a corresponding gene product in the host which is (1) dysfunctional, (2) in excess, or (3) appears in the wrong context or at the wrong developmental stage in the parasite's life cycle. If such faulty signals are designed specifically for parasitic cell functions, they will have little effect on the host. Therefore, resistance to a particular pathogen can be achieved by cloning the appropriate parasite gene, if necessary modifying its expression, and transforming it into the host genome. By resistance is meant any reduction in virulence of the parasitic infection or any reduction in the susceptibility of the host to the parasite.

This approach to engineering resistance has important advantages:
1) The source of resistance genes would never be in question, since each parasite would bring with it the genes necessary for deriving resistance.
2) The stability of parasite-derived resistance will generally be greater than the stability of simply inherited forms of host resistance, for reasons that are discussed later in more detail.
3) The difficulties involved in cloning genes from host organisms, which generally have larger genomes relative to their pathogens, are lessened.
4) Parasite-derived resistance will have a minimal effect on the host and should not produce substances harmful to man.

The general concept of parasite-derived resistance has not previously been developed despite the prior art previously discussed. There are several reasons for this. Firstly, the examples of negative interaction have generally been serendipitous observations of a natural effect and have been viewed in isolation, as special cases. Secondly, negative interactions have generally been regarded from an academic perspective with little consideration for practical applications. This is most clearly evident in the above-mentioned . papers on complimentary oligonucdeotides, wherein none of the authors make any connection with their own work and host/parasite relations or potential engineering of resistance. Also a recent review in a symposium on the use of anti-sense strands makes no mention of the application of this approach to host-parasite systems (Science, 1984, 225: 819). Thirdly, the general concept of parasite-derived resistance is based on a perspective other than the conventional wisdom. To the inventors' knowledge no one has deliberately engineered a parasite-derived resistance of any kind. Deliberateness is part of the claimed invention as indicated by language calling for "isolating" and "inserting" gene Fragments.

In addition, a review of the literature has revealed an absence of any prior art in the following specific areas:
1) Use in the host of viral genes coding for regulatory proteins, such as coat proteins, to interfere with viral reproduction.
2) Use in the host of derivatives of a viral replicase gene or a viral reverse transcriptase gene for the purpose of interfering with viral reproduction.
3) Use in the host of specific binding sites from the viral genome, for the purpose of interfering with viral reproduction.
4) Use of lysis genes derived from a parasite, to be used as inducible "self-destruct" genes for the engineering of hyper-sensitivity into the host.
5) Use in the host of anti-sense nucleic acid sequences derived from a parasite, for the purpose of interfering with parasite function and reproduction.
6) Use in the host of avirulence genes from a parasite, for the purpose of over-riding the virulence genes in virulent strains of the parasite.
7) Use of parasite genes involved in the biosynthesis of key regulatory molecules, for the specific purpose of engineering the same biosynthetic capabilities into the host, such that the host produces hormones, neurotransmitters, pheromones, or similar molecules which are disruptive to the parasite.

As an example of how disease resistance can be engineered by this approach, the discussion below sets forth in detail how the genes of the bacteriophage Q β can be used to make E. coli resistant to Q β infection. This example is not to be considered limiting of the invention but is an example of the ease with which the invention can be practiced, now that it is disclosed.

The biology of Q β and other RNA phages has been extensively documented (Zinder, 1975), and the cDNA sequence of its genome has been determined. The Q β genome has three major cistrons. These code for a maturation protein ( involved in lysis and phage binding to host pili), a coat protein, and a subunit of the replicase enzyme. (A fourth gene product is a minor coat protein which is a read-through product of the coat cistron.)

The life cycle of Qβ is basically as follows. The phage begins by binding to the sex pili of F' E. coli, through which it enters the cell and begins to translate its replicase subunit. Its replicase subunit polymerizes with three host subunits normally involved in host protein translation. The resulting hybrid tetrameric enzyme has RNA replicase activity specific for Q β. This specificity is due to the affinity between the Qβ subunit of the tetrameric replicase and a short segment of the Q β genome within the replicase cistron. The replicase attaches to Q β RNA at this binding site and replicates the viral RNA. Late in the life cycle of Q β, coat protein and maturation protein accumulate in the host. The coat protein then binds to the replicase cistron and thereby represses translation of the replicase subunit. Termination of replication allows viral assembly, and eventually the maturation protein lyses the host, releasing a new population of infective Qβ.

From a conventional (prior art) perspective, the life cycle of Q β suggests two potential mechanisms for developing resistance. Host-derived resistance might be developed by (1) blocking Q β binding to sex pili or (2) producing variant host subunits lacking affinity for the Q β replicase subunit. Blocking Q β binding is, in fact, a known mechanism for producing Q β resistance, since non-F' mutants lacking pili are immune to infection (Silverman, Rosenthal, Mobach and Valentine, 1968). However, this strategy clearly disrupts a mechanism which is relevant to the host's fitness as a species. The selection of variant forms of the host subunits which help make up the replicase enzyme may also be a naturally occuring mechanism conferring resistance. Since the host supplies 3 of the 4 subunits of the viral replicase, one might expect mutations within these genes to confer resistance. However, the extent to which these host subunits can be altered is clearly limited, since these subunits are essential to host protein synthesis and the survival of the host. Most of the variants of these host subunits would probably be lethal or sub-lethal for the host. Even non-lethal variants are likely to be suboptimal for protein translation efficiency. Therefore, both of the host-derived resistance mechanisms suggested by the Q β life cycle would be obtained at the expense of disrupting crucial host functions.

The prospect of being able to transfer genes from parasite to host provides a new approach to resistance. Viewed from this perspective, the life cycle of Q β suggests at least as many mechanisms of pathogen-derived resistance as host-derived resistance. Several strategies are seen to be promising: (1) deriving resistance from the Q β coat protein; (2) deriving resistance from a modified Qβ replicase; (3) deriving resistance by cloning the Q β replicase binding site, and (4) deriving resistance from expression of anti-sense strand RMA sequences. Another strategy involving the maturation protein also appears feasible.

Among the different strategies mentioned above, the one deriving the resistance from the anti-sense strand RNA sequences constitutes the object of this divisional application.

Anti-sense strand interference - The presence of an RNA complementary to Q β RNA would allow formation of an RNA-RNA duplex that would block Q β infection. This can be accomplished, for example, by transcribing a cDNA clone of a portion of Qβ in the reverse orientation in the E. coli host. The anti-sense strand RNA produced will then hybridize to the infecting Qβ and interfer with its proper translation or packaging. The advantages of this approach are that potentially any fragment of the viral genome could be used without modification, and it would he extremely difficult for the virus to overcome this form of resistance.

EP-A 140.308 describes regulation and inhibition of genetic expression of an organism by incorporating into the genetic material of said organism DNA or other genetic material which transcribes to an RNA which is complimentary to and capable of binding to the RNA of the genetic material of the organism.

Weintraub et al., TIG-January 1985, Vol. 1(1), 22-25, describes experiments showing that an endogenous gene is inhibited by anti-sense RNA and point out that many questions remain unanswered about the use of gene inhibition by using anti-sense RNA.

Although the two references indicate some potential uses of anti-sense RNA, there is no teaching from the above prior art that would suggest to use an anti-sense methodology to confer resistance to a host against an incoming parasite.

Although the examples set forth above describing methods by which bacteria can be protected from bacteria phage Q β are related in particular to a specific host/parasite system, the techiques are readily applicable to other systems, such as the protection of other organisms from both viral and non-viral infections. Techniques for achieving these results are set forth in more detail in the following paragraphs.

Virus resistance - The most likely early application of the concept of parasite-derived resistance is in engineering virus resistance. This is because the viral genome is small, and, since virus only propagates in the host, most of the genome is involved in pathenogenicity. Portions of the viral genome can be cloned and their potential for conferring resistance readily determined. Alternatively, resistance-conferring genes can be discovered empirically by testing the biological effect of various DNA restriction fragments of the viral genome. Most virus-derived resistances are likely to involve a block in replication. The methods described for engineering resistance to Q β are directly applicable to any virus which a) codes for a protein which helps regulate the virus' reproduction; b) has specific binding sites in its genome; c) synthesizes its own replicase or reverse transcriptase; or d) is bound by complementary reverse strand sequences of nucleic acid. In other words, these methods would apply to essentially all viruses.

While there has been some controversy among biochemists regarding whether plant viruses encode their own replicase, it now seems likely that most plant viruses do code for all or part of their replicases (Hall, Miller and Bujarski, 1982; Dorssers, Van der Meer, Kamen, Zabel, 1983). The first plant virus to have its replication mechanism characterized, turnip yellows mosaic virus, has proven analogous to Q β (Mouches, Candresse and Bove, 1984). This virus has been shown to to have a hybrid replicase, with its own sub-unit conferring specific binding to its genome. This indicates that the approach described for Qβ replicase would also apply to this virus. It is likely that most or all RNA plant viruses will code either for their own replicase, a subunit of the replicase, or a protein modifying the specificity of the host's RNA polymerase. This means that the replicase-derived resistance strategy outlined for Qβ will be directly applicable to a wide range of plant viruses. Many viruses have not yet been analyzed relative to this genetic structure. However, the very small size of the viral genome and the diversity of potential resistance mechanisms clearly indicates that a viral-derived resistance gene can be derived from any virus simply by using standard shotgun cloning methods and direct screening for subsequent resistance to the virus.

Non-viral resistance - The application of parasite-derived resistance to extracellular parasites is more complex than for viral parasites. Since false signals coded for by the host must be recognized by the parasite, parasite-derived resistance will only he useful where mechanisms exist which allow recognition or incorporation by the parasite of non-degraded macromolecules from the host. Van der Plank (1978) has offered persuasive theoretical arguments indicating that such an exchange of macromolecules between the host and the parasite often occurs. There is at least one case where such incorporation has been documented. In the malaria host/parasite system the parasite has been shown to incorporate and utilize a host dismutase enzyme, indicating the presence of a protein exchange mechanism (Fairfield, Meshnick and Eaton, 1983). To the extent that such mechanisms exist in other non-viral host/parasite relationships, the techniques described herein can be applied without significant modification. The existence of protein exchange mechanisms can be determined using monoclonal antibody probes to locate sub-cellular components, in conjunction with 2-D electrophoretic studies searching for host-parasite hybrid proteins.

Given a macromolecular exchange mechanism, a variety of approaches to the engineering of parasite-derived resistance exist for either viral or non-viral parasites. For example, in gene-for-gene host/parasite systems (Flor, 1971; common in viral, fungal, and bacterial pathogens), it is generally found that the parasite's avirulence alleles are dominant to virulence alleles (reviewed in Van der Plank, 1978). This suggests that the avirulence gene products override or block the activity of the virulence gene products - thereby preventing infection. Thus, an avirulence allele cloned from an avirulent strain of the parasite, when introduced and expressed constitutively in a transformed host, can enter the parasite or act at the host-parasite interface and override the infective capacity of an otherwise virulent pathogen. Avirulence alleles can be identified by a variety of methods. For example, in bacteria the virulence-avirulence locus can be cloned by using insertional mutation (employing transposable elements) of the virulent strain and screening for non-virulent mutants or by screening a genomic library for complementation of the virulence allele. The virulence gene can then be introduced into the host to confer resistance. Recently, an avirulence gene has been cloned from the bacterial pathogen Pseudomonas syringae. However, the expressed intent of these workers is to clone the resistance gene from the host and the parasite gene has not been introduced into the host in any form (Staskawicz et al 1984). The technique proposed here introduces an entirely new dimension to the classical model of gene-for-gene host/parasite interactions.

Resistance from the parasite's regulatory genes - A more general strategy for engineering parasite-derived resistance (applicable with or without gene-for-gene interactions) utilizes specific regulatory genes from the parasite. For example, fungal genes regulating haustorial development or sporulation can be introduced into a host, thereby disrupting the normal life cycle of the fungal pathogen, using established techniques of identifying the regulatory protein and searching a genomic library with an antibody probe. Once cloned, such genes can be introduced into'a host, where they will disrupt the normal life cycle of the fungal pathogen. This type of regulatory approach appears particularly useful in the engineering of insect resistance. For example, all insects depend on the regulated biosynthesis of juvenile and molting hormones for precise timing of molting, metamorphosis and reproduction. Using the techniques of this invention, it is possible to incorporate into the host genes from the insect pest encoding the activities necessary to produce the insects' hormones, pheromones or neurotransmitters. In the case of neurotransmitters, these polypeptides are typically extremely short (less than 20 amino acids) and are therefore easily sequenced, and artificial genes coding for these sequences can then be synthesized de novo. In the case of non-peptide hormones or pheromones the problem is more difficult but can be overcome. Typically, several enzymatic steps will be required from the starting point of a common precursor in both host and parasite to the biologically active secondary metabolite. This means that several genes in the parasite will have to be identified, cloned and transferred to the host. While this approach does not have the simplicity or directness of other parasite-derived approaches, it is potentially one of the more significant and broad-spectrum applications of parasite-derived resistance, and will generally warrant the time and expense of engineering the latter part of a biosynthetic pathway. The host producing such insect growth regulators or transmitters would be resistant by virtue of disrupting the behavior or life cycle of the insect pathogen, thereby eliminating infection of the primary host. There are examples in nature where plants seemed to have exploited a similar strategy for resistance by evolving genes producing analogs to, or biosynthetic antagonists of, insect hormones (Bowers, 1980).

Another application of parasite-derived resistance is available where an insect or other organism serves as an intermediate host, so that the disease cycle can be disrupted by making the intermediate host resistant to the pathogen, thereby eliminating infection of the primary host. For example, efforts to control malaria have previously focused on eradication of the intermediate host, tile Anopheles mosquito. If, however, genes from the Plasmodium pathogen are introduced into the mosquito in a manner to confer resistance by disrupting the life cycle of the parasite, the disease cycle will he broken. This approach is most feasible if the resistance genes is of selective advantage to the intermediate host, allowing resistance genes to be maintained and propagated in natural populations after introduction of modified individuals. This can be done, for example, by concurrently introducing resistance to a pesticide into the intermediate host.

### Advantages and Limits of Pathogen-derived resistance

Parasite-derived resistance represents a systematic and broadly-relevant approach to the problem of how to genetically engineer insect and disease resistance. The rich possibilities of this approach are illustrated by the fact that three different strategies for deriving resistance from the Qβ bacteriophage exist in a parasite having only three genes. There are several distinct advantages of parasite-derived resistance.

One of the most attractive features of parasite-derived resistance is that each new parasite or race of parasite that becomes a problem simultaneously brings with it the specific genes needed to engineer resistance to itself. These genes can be systematically identified within the parasite's genome. Once such genes have been identified, homologous genes in other parasite races or in related parasites will be readily identifiable by DMA hybridization techniques. This eliminates the need for repeated and exhaustive searches through the host's germplasm pools, seeking rare host resistance genes.

Another major advantage of this strategy is that it should not generally be disruptive of host functions. Van der Plank (1978), using evolutionary arguments and population genetics data, has argued that host genes controlling susceptibility exist because they involve essential host functions. Most hosts are genetically susceptible because the susceptible allele is optimal relative to its natural function. Host-derived resistance alleles, therefore, tend to disrupt the optimal functioning of the host. To the extent that this is true, most host-derived resistances attack the pathogen indirectly by replacing an optimal host gene product with a non-optimal host gene product which happens to be incompatible with the parasite. This is seen in the Q β system, where host-derived resistance is likely to be achieved either by disrupting sex pili formation or by tampering with the host's protein-synthesis machinery. A similar situation exists with sickle-cell anemia, which is harmful to humans when expressed but which confers resistance to malaria in persons who have both a recessive sickle-cell gene and a normal hemoglobin gene. The beauty of the concept of pathogen-derived resistance is that only pathogenic cell functions are attacked and are attacked directly, which will have minimal subsequent effect on the host. The specificity of parasite-derived resistance is not only desirable in terms of being non-disruptive to the host, but also of being non-harmful to man. Resistance based upon production of general toxicants, such as the natural pesticides of many resistant plant taxa, have been shown to be potentially harmful to man when ingested (Ames, 1983). The specificity of parasite-derived resistance should preclude, to a large extent, any such harm to man.

There are reasons to believe that parasite-derived resistance should be relatively durable compared to host-derived resistance. The ability of parasites to circumvent host-generated general toxicants is well known. Additionally, specific host-derived resistance genes are frequently overcome by matching gene-for-gene mutations to virulence in the parasite (Flor, 1971). In the case of host-derived Q β resistance, alterations in the host replicase sub-units (making them incompatible with the viral subunit, thereby conferring resistance), are easily matched by mutations in the Q β replicase subunit which restore subunit compatability, constituting a mutation to virulence. However, such gene-for-gene mutations circumventing resistance should be relatively rare in the case of parasite-derived resistance. In this case the parasite would usually be facing a new form of resistance, which it had not previously faced in its evolution. These types of resistances are likely to be very difficult for the parasite to overcome, especially where regulatory genes are involved. For example, if resistance to Qβ was derived from the Qβ coat protein gene, a new virulent Qβ strain could only arise by first having a new binding site develop by mutation in the replicase cistron (without disrupting replicase function) which would not bind the native coat protein. Simultaneously a new coat protein would have to arise by mutation (without disrupting coat protein function) which would bind to the new binding site. Such a simultaneous and complementary set of mutations (which preserved both coat and replicase functions) should be extremely rare.

Last, engineering parasite-derived resistance should be considerably more approachable on the molecular level 1 than engineering host-derived resistance. There are numerous reasons for this: (1) this strategy would generally focus on the molecular biology of relatively simple organisms with short life cycles; (2) it would generally require only the identification and isolation of individual genes from small genomes; (3) unregulated, constitutive expression of the parasite-derived resistance genes would usually be effective; and (4) it would avoid the complex, multigenic biosynthetic pathways which are the likely basis of many existing host-derived resistances.

There do not seem to he any obvious disadvantages to the parasite-derived approach to resistance, except that application of the strategy to non-virus parasites is only possible where mechanisms exist for macromolecular exchange between host and parasite. Most forms of parasitism, especially those forms displaying gene-for-gene resistance, allow ample opportunity for gene-product interactions and will be suitable for engineering parasite-derived resistance.

Techniques for the production of resistant host - As will be readily understood that those of ordinary skill in the art of genetic engineering, standard techniques of genetic engineering can readily be adopted to attain the goals set forth herein. Protection of a host against a virus, for example, can easily be achieved. Because of the reasons set forth above, it is not necessary to identify the gene being inserted into the host, although identification of the gene will make application of the method easier to perform. In general, genetic information (DNA or RNA) from any virus is isolated using standard procedures and cleaved into pieces of varying lengths, preferably containing at least 20 nucleotides if the DNA is to be transcribed in an anti-sense direction, or at least a functional portion and preferably an entire gene if the gene is to be expressed. DNA fragments are typically obtained using restriction endonuclease enzymes. The same enzyme (or enzymes) is then used to cleave a vector capable of replicating in the host or inserting into a host's chromosome. The vector can be a natural plasmid or transposon or any part thereof capable of replication in the host and, when desired, production of a gene product from the exogenous parasite gene fragment. Vectors derived from plasmids and other vectors normally present in the host are preferred. The viral DNA is inserted into the vector using standard techniques in either a sense direction (when expression of a gene product is desired) or an anti-sense direction. Proper tailoring of the gene fragment in the vector (e.g., employing appropriate 5' and 3' flanking sequences to ensure regulation, transcription, and translation as desired) is readily achieved using standard techniques, especially when simple constitutive expression is desired, as is suitable in most cases of parasite-derived resistance. As used in this application, the phrase "gene fragment" encompasses both entire genes, DNA segments that contain an entire gene or a portion thereof, and segments of DNA that are incomplete parts of a single gene. The word "gene" encompasses both DNA sequences that code for a peptide gene product and other DNA sequences that form a functional part of a chromosome or plasmid.

Although this specification generally refers to DNA alone when describing genetic information, vectors, or the like, this is done for ease of expression only. Any reference to DNA, unless clearly restricted to DNA and not to RNA, is equally applicable to RNA. For example, pathogenic RNA viruses can be the source of the parasite gene fragment, and non-virulent RNA viruses can act as vectors. In many instances, however, it is easier to work with DNA than RNA (e.g., more DNA restriction endonuclease enzymes are known), and use of cDNA prepared from RNA is a preferred embodiment of the invention when producing resistance to an RNA virus.

After a gene fragment has been isolated, the DNA sequence can be determined and modified, if desired, to produce similar DNA segments capable of being expressed as the same or similar gene products. For example, one or more codons can be replaced by equivalent codons to produce artificial DNA segments coding for the identical gene product. Alternately, a codon can lie replaced by a codon that codes for a similar amino acid (e.g., a codon for lucine replaced by a codon for isoleucine or a codon for glutamic acid replaced by a codon for aspartic acid). When used as an antisense strand or binding site, less than 10% non-identical nucleotides are preferred with unmodified gene fragments being most preferred. Greater modification of the gene fragment is possible when a gene product of the parasite gene is being produced. For example, artificial DNA sequences containing a series of codons functionally equivalent (i.e., that code for the same amino acids) to the codon sequence in the parasite gene fragment are considered fully equivalent to the parasite gene fragment since they will produce the same gene product, even though the DNA sequence can be substantially different. Gene products not identical to the natural gene product but retaining the ability to produce a gene product capable of disrupting an essential activity of the parasite can be produced by systematic modification of codons (and thus the expressed gene products) followed by testing for parasite resistance. Such modified DNA segments must be substantially homologous to at least a part of the isolated gene fragment or a DNA sequence functionally equivalent thereto in order to be considered indicative of parasite-derived resistance. By "substantial homology" is meant at Least 80%, preferably at least 90%, and most preferably at least 95% identity between the DNA sequence in question and the sequence to which it is being compared. Identical sequences are also covered by the same phrase. Comparisons for the purpose of determining homology are preferably made over a sequence of at least 15 and more preferably at least 21 nucleotides.

The phrase "isolating a gene fragment", as used in this application, refers to the process of obtaining a gene fragment to be used in the production of resistance in a useful form. The gene fragment does not have to be purified or otherwise separated from other cellular components, although this will occur in many processes. Instead, the word "isolated" is used to indicate that a gene has been obtained in a useful form by a deliberate process. For example, an "isolated gene fragment" can exist in a mixture of fragments from the DNA of a parasite that is to be used in a shotgun cloning procedure. A gene fragment is also still "isolated" when it is present in the form of a recombinant plasmid present in a bacterium being used in a shotgun cloning procedure to identify producers of desired parasite gene products (such as by use of monoclonal antibodies). Likewise, a segment of purified DNA comprising a parasite gene segment and termini from a cloning vector (e.g., obtained by cloning a parasite gene fragment in a bacterial plasmid prior to insertion into the final host) is also encompassed by this term. Other usable forms of gene fragments will be readily apparent to those skilled in genetic engineering.

Insertion of the parasite gene fragment into a host is readily achieved when the host is a bacterium or other unicellular organism since the major advances that have occurred recently in genetic engineering have generally involved insertion of vectors containing exogenous genes into unicellular hosts (especially bacteria and yeasts) and are directly applicable to the present method. "Insertion" encompasses any means of introducing genetic information into a host organism compatible with the limitations discussed in this specification. However, insertion in a manner to provide a heritable characteristic is preferred. In unicellar organisms this can readily be accomplished using heritable plasmids or by insertion of the parasite gene fragment into the host chromosome. These examples are not limiting, and other methods of inserting heritable genetic information, whether into unicellar or higher organisms, are equally applicable to the practice of this invention.

Proven methods for inserting new genes into higher organisms can now be found in a massive volume of current literature. There exist four basic methods of doing this (Baserga, Crose, and Povera, Eds., 1980) : (1) direct uptake of DMA or DNA-containing particles by the cell, (2) cell fusion with other cells or ghost cells, (3) microinjection, and (4) infective transformation. A fifth method is being developed which involves tile use of accelerated high-velocity one-micron-sized particles for the purpose of carrying DNA into cells and tissues.

Uptake mechanisms include the following:
(1) induction of enhanced membrane permeability by use of Ca⁺⁺ and temperature shock (Mandel and Higa, 1970; Dityakin et al., 1972); (2) use of surface binding agents such as PEG (Chang and Cohen, 1979; Krens et al., 1982) or Ca(PO₄)₂ (Graham and van der Eb, 1973; Wigler et al., 1979); and (3) phagocytosis of particles such as liposomes (Uchimaya et al., 1982), organelles (Potrykus, 1973), or bacteria (Cocking, 1972), into the cell. These uptake mechanisms generally involve suspensions of single cells, where any existing cell wall materials have been removed enzymatically. Uptake protocols are generally quite simple and allow treatment of large numbers of cells en masse. In such systems most cells are unaffected, but cell selection procedures are available to recover the rare cells that have been transformed (Powers and Cocking, 1977).

Fusion mechanisms incorporate new genetic material into a cell by allowing it to fuse with another cell. A variation on this theme involves ghost cells. The membrane of killed cells are allowed to fill with a given DNA solution, such that cell fusion incorporates the DNA from the carrier "cell" into the living cell. Cell-to-cell fusion can be induced with the aid of such things as PEG (Bajaj, 1982) and Sendai virus particles (Uchida et al., 1980). As with uptake mechanisms, fusion technologies rely upon the use of single cell suspensions, where cells are enzymatically stripped of any cell wall material. While fusion technologies can have relatively good efficiencies in terms of numbers of cells affected, the problems of cell selection can be more complex, and the resulting cells are typically of elevated ploidy.

Microinjection technologies employ extremely fine, drawn out capillary tubes, which are called microelectrodes. These can be made sufficiently small that they can be used as syringe needles for the direct injection of biological substances into certain types of individual cells (Diacumakos, 1973; Graessmann and Graessmann, 1983). One modification of microinjection involves pricking with a solid-glass drawn needle, which carries in biological solutions which are bathing the cell (Yamomoto et al., 1981). Another modification is called ionophoresis (Purres, 1981; Ocho et al, 1981), which uses electrophoesis of substances out of the microelectrode and into the cell as an alternative to high pressure bulk flow. Microinjection procedures can give extremely high efficiencies relative to delivery into the cell. Because of this, microinjection has been used successfully in the transformation of individual egg cells.

In another example, foreign DNA was successfully injected into cotton pollen tubes without the pollen being damaged or its germination being inhibited. Although this involved a resistance gene from another plant instead of a parasite gene, the same technique can be used in the practice of the present invention. DNA was injected into the nucleus of cotton pollen grains germinating on cellophane using micro-manipulators and a micro-injection system. This operation was carried out on the fixed stage of an inverted research microscope equipped with Nomarski differential interference optics. Foreign DNA in a recipient nucleus was detected by epifluorescence after the incorporation of a fluorescent marker in the injected material. The DNA was introduced using "quickfill" tubing drawn to a tip diameter of 0.5 micron, and the DNA was injected into the nucleus iontophoretically. The germinating pollen was returned to the style where it continued to grow and fertilize the ovule. About 20 injections can be carried out per day. Seeds from the micro-injected plants were planted, and seedlings were raised and screened. Screening nay be carried out by testing for the presence of the foreign gene by Southern blotting or for the presence of the gene product by means of enzyme inhibition assays. In addition, screening for insect resistance of the developing square and boll can be utilized when cotton is the host. Other plants can be treated in the same manner.

Infective transformation employs non-injurious infective agents of the host, such as viruses, which naturally transmit part of their genome into the host. In plants, the principal mode of transformation now being practiced is the use of the infective agent Agrobacterium tumefaciens. This bacterium will naturally colonize cells of any dicotyledonous plant and transmit a specific "T-region" of its Ti-plasmid into the plant chromosome. Other plant vectors useful for the transformation of plants can similarly be used. Genes of interest can now be routinely engineered into the T-region and can be transmitted to the plant by the bacterium (see Fraley et al., 1983). Simple conincubation (growing plant cells and bacterial cells together) has been shown to be extremely effective in transforming plant protoplasts and leaf disks, and whole transformed plants have now been regenerated in numerous plant species (see Horsch et al., 1984). In mammals, naturally infective retroviruses have been used to construct naturally transforming vectors which insert engineered DNA into the mammalian chromosome, in a manner similar to Agrobacterium tumefaciens. This transformation mechanism is considered extremely promising for animal and human gene therapy (see Anderson, 1984).

For an example of mammalian transformation, see U.S. Patent 4,396,601 to Salser et al., which describes a technique in which cells are isolated from a regenerative body member of a mammal or a syngeneic equivalent to provide parent cells. The parent cells are combined with DNA from the parasite and with additional DNA that produces a a selection advantage over the parent cells when the cells are subjected to mitotic inhibition. The modified cells are then introduced into the host in a manner such that the modified cells return to the body member from which the parent cells were obtained. A mitotic inhibitor is then administered to the host to provide a selective advantage for the modified cells over the parent cells, thereby regenerating the modified cells in the host. Further details of this method can be obtained by reference to U.S. Patent 4,396,601.

The method of the invention is generally applicable to the protection of any host from a parasite of that host. As used herein, "host" refers to any organism that can be infected by any parasitic or symbiotic organism. The term "parasite" refers to any organism that obtains substance or means for reproduction from an organism, whether it lives with that organism in a parasitic or symbiotic relationship. The parasite need not be specific for a particular host but may be a parasite of many hosts, such as the caterpillars of numerous moths and bufferflies. Although a preferable parasite for use in this invention is a virus, whether the virus is a DNA or RNA virus, other parasites are also encompassed by this term. Examples of other parasites include bacteria, protozoa, fungi, nemotodes, insects, and arachnids.

Since a host is normally higher in the evolutionary scheme than the parasite, the term "host" does not encompass a virus, which resides at the bottom of the evolutionary scheme. However, any higher organism is capable of being infected by a parasite. The invention is readily applicable, for example, to bacteria grown in culture which need protection against infection from bacteriophages. Additionally, plants and other higher organisms, such as mammals, also can be readily protected from viruses using the method of the invention. Both plants and animals can also be protected from higher parasitic hosts, such as insects and protozoans, subject to the restrictions which have already been discussed. Examples of hosts include bacteria, yeasts, fungi (e.g., mushrooms), legumious plants (e.g., soybeans), cereal and forage crops (e.g., corn, wheat, rice and alfalfa) , food crops (e.g., tomatoes, potatoes, lettuce, and onions), ornamental plants (e.g., roses, junipers, and orchids), trees (e.g., pine, spruce, and walnut), protozoans, amphibians, reptiles, birds (e.g., chickens and turkeys), and mammals (e.g., cats, dogs, horses, cattle, sheep, goats, pigs, and primates).

Examples of host/parasite systems in which either the host or the parasite is a unicellular organism (the most common situations) can be found in numerous microbiology textbooks and reference manuals, such as CRC Handbook of Microbiology, Condensed Edition, Laskin and Lechevalier (eds.), CRC Press, Cleveland, Ohio, 1974. Other examples of host/parasite systems are given below along with examples of how resistance to the parasite can be given to the host in that system. These examples are not limiting, and many other methods for achieving resistance are possible for each listed system.
1) There are a variety of bacteria important in industrial fermentation processes, such as Streptococcus lactis, Streptococens cremoris, and Lactobacillus species. During fermentation, infection by various bacteriophages is a common cause of fermentation failure. Bacterial resistance to such bacteriophage infection can be engineered by methods exactly analogous to the methods described above for engineering resistance to the Q β bacteriophage in E. coli.
2) There are hundreds of significant plant RNA viruses, and essentially all crop species are affected by one or more such viruses. Resistance to such viruses can be obtained in a manner closely analogous to Q β resistance in bacteria, by cloning fragments of the viruses into plant-transforming vectors such as a modified Ti-plasmid and transforming the appropriate plants. Plants transformed by various gene tragments can then be screened for resistance, using established plant breeding techniques. A few relevant viruses include alfalfa mosaic virus, brome mosaic virus, barley yellow dwarf virus, beet yellows virus, cucumber mosaic virus, lettuce necrotic yellows virus, maize chlorotic dwarf virus, pea enation virus, potato viruses S, X, and Y, southern bean mosaic virus, tomato ringspot virus, tobacco ringspot virus, tobacco mosaic virus, tobacco streak virus, turnip yellow mosaic virus, and wound tumor virus.
3) There are certain animal and human pathogens, such as the flu and common cold viruses, which have evolved mechanisms for circumventing the effectiveness of the animal immune system. Where such a virus is a chronic problem, as with flu and colds, parasite-derived resistance will be a powerful tool for conferring immunity to all strains of that pathogen. Resistance can he engineered by cloning fragments of the viral genome, introducing the gene fragments into animal cells in vitro by use of retroviral vectors, testing of varius tranformed cell times to determine which have conferred resistance to infection by the virus, and then using those fragments conferring resistance to create benign non-infectious retrovirus vectors for the purpose of introducing resistance genes into individuals.
4) There are certain retroviruses which attack T-cells (i.e., the human immune system) directly (such as the viruses that produce AIDS), thereby circumventing our natural immune defense mechanism. Resistance can be engineered as described above, using AIDS genomic fragments, and also using AIDS, or a similar retrovirus, for the construction of a T-cell-specific transforming vector. Transformed T-cells with resistance-conferring fragments of the AIDS genome would have a selective advantage over other susceptible T-cells, becoming the predominant form of T-cell and thereby giving rise to resistant individuals.
5) A wide range of bacteria and fungi that parasitize plants have intimate contact with living host cells and reveal gene-for-gene host parasite relations. Resistance in such cases can be engineered by cloning avirulence alleles from avirulent strains of the parasite and introducing these genes into the relevant host for the purpose of conferring resistance. A few pathogens where this method is relevant include Puccinia sorghi infection of corn, Puccinia infections of wheat, Phytophthora infestans infection of potato, Ustilago infection of rye, and Melampsora lini infection of flax.
6) A wide range of insects parasitize plants, causing severe economic losses, and depend upon a proper balance of juvenile hormone and molting hormone to regulate their development. Therefore, broad-spectrum, insect-derived plant resistance to insects can be engineered by cloning the insect genes responsible for the final steps of the biosynthesis of these hormones and transferring these genes to the plant hosts of interest, using established transformation techniques. Typical genes would code for enzymes controlling the conversion of a precursor into the desired regulatory product (e.g., hormone). Basically all plant hosts contain the precursors for the synthesis of these hormones; i.e., farnesol in the case of juvenile hormone and phytosterols in the case of molting hormone. Other useful genes would be those producing other regulatory substances that trigger the production of hormones in parasites. A few insect parasites which could be controlled by this method include flea beetles, wire worms, cutworms, grubs, aphids, leafhoppers, tarnished plant bugs, Colorado potato beetles, cucumber beetles, weevils, cabbage worm, cabbage lopper, leafminers, Hessian fly, grasshopper, tent worm, gypsy moth, tussock moth, army worm, corn ear worm, European corn borer, and Japanese beetle.
7) A wide range of insects parasitize plants and contain neurotransmitters which control essential body functions. Such neurotransmitters are oligopeptides typically only 5-20 amino acids long. In this case insect-derived resistance can be engineered by sequencing the oligopeptide and synthesizing artificial genes homologous to the native insect genes coding for these neurotransmitters. These synthetic genes, when expressed in a plant host, can then disrupt that crucial body function normally regulated by that neurotransmitter of the insect parasite. The insect listed in the previous example would be equally valid as candidates for this method of deriving parasite-derived resistance.

In addition to the above general procedures which can be used for preparing recombinant DNA molecules and transformed unicellular organisms in accordance with the practices of this invention, other known techniques and modifications thereof can be used in carrying out the practice of the invention. In particular, techniques relating to genetic engineering have recently undergone explosive growth and development Many recent U.S. patents disclose plasmids, genetically engineered microorganisms, and methods of conducting genetic engineering which can be used in the practice of the present invention. For example, U.S. Patent 4, 273, 875 discloses a plasmid and a process of isolating the same. U.S. Patent 4,304,863 discloses a process for producing bacteria by genetic engineering in which a hybrid plasmid is constructed and used to transform a bacterial host. U.S. Patent 4,419,450 discloses a plasmid useful as a cloning vehicle in recombinant DNA work. U.S. Patent 4,362,867 discloses recombinant cDNA construction methods and hybrid nucleotides produced thereby which are useful in cloning processes. U.S. Patent 4,403,036 discloses genetic reagents for generating plasmids containing multiple copies of DNA segments. U.S. Patent 4,363,877 discloses recombinant DNA transfer vectors. U.S. Patent 4,356,270 discloses a recombinant DNA cloning vehicle and is a particularly useful disclosure for those with limited experience in the area of genetic engineering since it defines many of the terms used in genetic engineering and the basic processes used therein. U.S. Patent 4,336,336 discloses a fused gene and a method of making the same. U.S. Patent 4,349,629 discloses plasmid vectors and the production and use thereof. U.S. Patent 4,332,901 discloses a cloning vector useful in recombinant DNA. Although some of these patents are directed to the production of a particular gene product that is not within the scope of the present invention, the procedures described therein can easily be modified to the practice of the invention described in this specification by those skilled in the art of genetic engineering.

All of the patents and other publications cited in this specification are indicative of the level of skill and knowledge of those skilled in the arts to which the present invention pertains.

In addition to the method of producing resistance to a parasite described above in detail, this invention also encompasses hosts produced by the process of the invention as well as recombinant vectors and other products of genetic engineering useful in the practice of the invention.

The invention now being generally described, the same will be better understood by reference to certain specific examples which are included herein for purposes of illustration only and are not intended to be limiting of the invention or any embodiment thereof, unless so specified.

### EXAMPLE

The feasibility of the concept outlined above was proven with experiments using the bacteriophage Q β and its host, E. coli. Using cDNA clones of Q β ( Q β is an RNA phage), plasmids were first constructed or obtained which would express part of the Q β cDMA in E. coli and confer resistance.
Coat Protein: The plasmid used for production of the coat protein was pGL101 obtained from R. !3. Winter (Cell 33, 977). This plasmid expresses the coat protein under lac operator control, so its expression can be induced by IPTG (though there is also a constitutive expression). This plasmid as well as the others described below contain the gene encoding amp^{r}.
Negative Strand: A plasmid was constructed that inserted the 0.9 Kb HpaII fragment of Q β cDNA into pUR222 plasmid at the AccI site. This fragment extends from positions 2547 to 3473 in Q β (FIGURE) and includes translational sequences of the replicase gene. These sequences also contain the M-binding site of the replicase. In the "sense" orientation of this fragment, a fusion product between β-galactosidase protein and the replicase fragment is formed. In the antisense (reverse) ligation of this fragment, an RNA complementary to the Q β RNA sequence is formed. Both constructions were made.
Testing for Resistance: The strain GMl (provided by R. W. Webster) was transformed with pUR222 or one of the test plasmids described above. These strains were grown up, made competent, incubated with Q β and then plated out in soft agar. Plaque numbers and sizes were assessed to determine if resistance was taking place.

In an initial experiment to test the coat protein, GMl + pUR222 and GM1 + pGL101 were grown in 10 mls L-broth containing ampicillin in [PTG. At stationary phase the cultures were pelleted and resuspended in 4 ml 50 mM YCaCl₂. A small portion, 0.1 ml, of this plating culture was incubated 60' with 10⁷ pfu of Q β. This was then plated on YT-AMP plates in 3 ml soft agar with IPIG. The results were that the GM1 + pUR222 plates had thousands of plaques which soon (24 hrs) engulfed the plate; the GM1 + pGL101 plate at first showed no plaques but later developed many very small plaques.

To check the possibility that the GMl strain + pGL101 resistance was due to loss of the F' element, the strains were subsequently grown on minimal medium lacking proline to maintain selection for the F'. The same protocol as above was then repeated, including strains of GM1 with the HpaII (sense) and HpaII (antisense) bearing plasmids. The results are presented in Table 1. Both the coat protein and the HpaII (antisense) plasmids could confer resistance to Q β infection. This experiment was repeated twice with essentially the same results. After continued passage, however, the plasmid bearing Q β cDNA sequences rearranged or were lost.

**Table 1**

| Strain + | Plasmid | Inducer | # of Plaques | Size |
|---|---|---|---|---|
| G11 | -- | + | 300 | normal |
| | puR222 | - | 360 | " |
| | " | + | 348 | " |
| | Hpa (antisense) | - | 247 | " |
| | " | + | 263 | small |
| | Hpa (sense) | - | 224 | normal |
| | " | + | 234 | " |
| | pGL101 | - | 176 | very small |
| | " | + | 101 | very very |
| small | | | | |

### References Cited

Ames, B.N. (1983). Science 221: 1256-1264.
Anderson, W.F. (1984). Science 226: 410-409.
Bajaj, Y.P.S. (1982). In: Reinert, J. and Bajaj, Y.P.S. (eds.), Plant Cell, Tissue and Organ Culture. Springer-Verlag, New York, pp. 467-496.
Baserga, R., C. Crose, G. Rovera, Eds. (1980). Introduction of macromolecules into viable mammalian cells. In: Sinstar Symposium Series VI. A.R. Liss Inc. New York.
Bowers, W.S. (1980). In: Insect Biology in the Future p. 613. New York, Academic Press.
Chang, S. and Cohen, S.N. (1979). Mol. Gen. Genet. 168: 111-115.
Cocking, E.C. (1972). Ann. Rev. Plant Physiol. 23: 29-50.
Diacumakos, E.G. (1973). In: Prescott, D.M. (ed.), Methods in Cell Biology. Academic Press, New York, pp. 287-311.
Dityatkin, S.Y., Lisovskaya, K.V., Panzhava, N.N., Iliashenko, B.N. (1972). Biochimica et Biophysica Acta 281: 319-323.
Dorssers, L., Meer, J. van der, Kammen, A. van, and Zabel, P. (1983). Virology 125: 155-174.
Fairfield, A.S., S.R. Meshnick, and J.W. Eaton. (1983). Science 221: 764-766.
Flashinan, S.F. (1978). Mol. Gen. Genet. 166: 61-73
Flor, H.H. (1971). Ann. Rev. Phytopathol. 9: 27S-296.
Fraley, R.T., et al. (1983). Proc. Natl. Acad. Sci. 80: 4803-4807.
Gibbs, A. (1969). Adv. Virus Res. 14: 263-327.
Graessmann, M. and Graessmann, A. (1983). Methods in Enzymology 101: 482-492.
Graham, F.L. and van der Eb, A.J. (1973). Virology 52: 456.
Hall, T.C., W.A. Miller, and J.J. Bujarski. (1982). In: Advances in Plant Pathology. P. 179. (Vol. 1) Academic Press, New York.
Hamilton, R.I. (1980). In: Viruses. Plant Disease: An Advanced Treatise. P. 279. (Vol. 5). Academic Press, NY.
Held, G.A., L.A. Rulla, E. Ferari, J. Hoch, A. I. Aronson, and S.A. Minnich (1982). Prov. Natl. Acad. Sci. 79: 6065-6069.
Horsch, R.B., R.T. Fraley, S.G. Rogers, P.R. Sanders, A. Lloyd, N. Hoffmann (1984). Science 223: 496-498.
Izant, J. and Weintraub, H. (1984). Cell 36: 1007-1015.
Krens, F.A., Molendijk, L., Wullems, G.J., and Schilperoort, R.A. (1982). Nature 296: 72.
Mandel, M. and Higa, A. (1970). J. Mol. Biol. 53: 159-162.
Marcus, P.I. and Carrier, D.H. (1967). J. Virology 1: 334.
Marcus, P.I. and Zuckerbraun, H.L. (1969). In: The Biology of Large RNA Viruses. P. 455. Acad. Press, New York.
Marcus, P.I. and Zuckerbraun, H.L. (1970). Ann N.Y. Acad. Sci. 173: 185-198.
Mizuno, T., Chou, M. and Inouye, M. (1984). Proc. Natl. Acad. Sci. USA 81: 1966-1970.
Mouches, C., Candresse, T. and Bove, J.M. (1984). Virology 134: 78-90.
Ocho, M., Nakai, S., Tasaka, K., Watanabe, S., and Oda, T. (1981). Acta Med. Okayama 35 (5): 381-384.
Potrykus, I. (1973). Z. Pflanzenphysiol. 70: 364-366.
Power, J.B. and Cocking, E.C. (1977). In: Reinhert, J., and Bajaj Y.P.S. (eds.) Plant Cell, Tissue and Organ Culture. Springer-Verlag, New York, pp. 497-505.
Purres, R.D. (1981). Academic Press, New York, 146 p.
Roberts, T.M., Kacich, R. and Ptashne, M. (1979). Proc. Natl. Acad. Sci. USA 76: 760-764.
Silverman, P.M., Rosenthal, S., Mobach, H., and Valentine, R.C. (1968). Virology 36: 142.
Staskawicz, A.J., D. Dahlbeck, and N. Keen (1984). Proc. Natl. Acad. Sci. USA 81:6024.
Uchida, T., Yamaizumi, M., Makada, E., Okada, Y. (1980). In: Introduction of macromolecules into viable mammalian cells. Windsor Symposium Series VI, A.R. Liss Inc., New York, pp. 169-185.
Uchimiya, H., Ohgawara, T., and Harada, H. (1982). In: Fujiwara A. (ed.), Proc. 5th Intl. Cong. Plant Tissue and Cell Culture, Jap. Assoc. for plant Tissue Culture. Tokyo. pp. 507-508.
Van der Plank, J.E. (1978). Genetic and Molecular Basis of Plant Pathogenesis. Springer-Verlag, NY. pp. 167.
Wigler, M., Sweet, R., Sim, G.K., Wold, B., Pellicer, A., Lacy, E., Maniatis, T., Silverstein, S., and Axel, R. (1979). Cell 16: 777.
Yamamoto, F., Furusawa, M., Furusawa, I., and Obinata, M. (1982). Exp. Cell Res. 142: 79-84.
Zinder, N.D. (ed) (1975). RNA Phages. Cold Springs Harbor Laboratory, NY pp. 428.
Barton, K.A., and Brill, W.J. (1983). Science 219: 671-676.
Illmensee, W. (ed.) (1984). Long Term Goal's in Agricultural Plant Improvement. Genetic Manipulation - Impact on Man and Society. Cambridge Univ. Press.
Day, P.R., Barrett, J.A., and Wolfe, M.S. (1983). Evolution of host-parasite interaction. In: Genetic Engineering of Plants: An Agricultural Perspective. Kosuge, P., Meredith, C.P., and Hollaender, A., eds. Plenum Press, New York.

## Claims

1. A method for conferring resistance in a non-human, non-animal host to a parasite thereof, which comprises:
(a) isolating a gene fragment from said parasite, and
(b) inserting in a manner to provide a heritable characteristic said gene fragment or a DNA or RNA segment substantially homologous to at least a part of said gene fragment or to a DNA or RNA sequence functionally equivalent to said gene fragment into said host,
wherein said gene fragment, or said DNA or RNA segment in said host is transcribed in an anti-sense direction, and the transcription product of said gene fragment or said DNA or RNA segment interferes with parasite functions and reproduction.

2. The method of Claim 1, wherein said inserting comprises transforming the host with a vector carrying said gene fragment where said vector is capable of stably replicating in the host or said gene fragment is capable of being inserted into a chromosome of said host.

3. A method as in claim 1 or 2 wherein the host is a plant or bacterium and the parasite is a virus.

4. The method of claim 2, wherein said host is a unicellular organism and said parasite is a virus capable of infecting said unicellular organism.

5. The method of Claim 2, wherein said host is a bacterium and said parasite is a virus capable of infecting said bacterium.

6. The method of Claim 2, wherein said vector is a plasmid or transposon found in said host or a portion of said plasmid or transposon capable of functioning as a vector.

7. The method of Claim 2, wherein said host is a plant and said parasite is a virulent virus capable of infecting said plant.

8. The method of Claim 7, wherein, said vector is a plasmid, transposable element or a non-injurious virus found in said host or a portion of said plasmid, transposable element, or virus capable of infecting said plant.

9. The method of Claim 7 wherein said vector is a Tiplasmid of Agrobacterium tumefaciens or a part thereof capable of functioning as a vector for the transformation of a plant.

10. The method of Claim 1 wherein said parasite is a virulent virus, fungus or bacterium having gene-for-gene host parasite relations, and said gene is a dominant avirulence allel from an avirulent strain of said parasite.

11. The method, of Claim 1 wherein said gene is a regulatory gene for said parasite or is a gene involved in the biosynthesis of a regulatory molecule in said parasite.

12. The method of Claim 1, wherein said regulatory gene codes for a gene product involved in the synthesis of an insect juvenile or molting hormone, an insect neurotransmitter, a fungus haustorial development or sporulation regulator, or a pheromone.

13. The method of Claim 1, wherein said host comprises plant cells.

14. Isolated Cells of an animal host which are resistant to a parasite of said animal host, wherein a gene fragment isolated from said parasite or a DNA or RNA segment substantially homologous to at least part of said gene fragment or to a DNA or RNA sequence functionally equivalent to said gene fragment has been inserted in a manner to provide a heritable characteristic, characterized in that the gene fragment or a DNA or RNA segment substantially homologous to at least part of said gene fragment or to a DNA or RNA sequence functionally equivalent to said gene fragment, which is inserted in a manner to provide a heritable characteristic into said cells of animal host, is transcribed in anti-sense direction and its transcription product interferes with the parasite functions and reproduction.

15. Isolated Cells of an animal host of claim 14 whereby the gene fragment or a DNA or RNA segment substantially homologous to at least part of said gene fragment or to a DNA or RNA sequence functionally equivalent to said gene fragment, which is inserted and transcribed in anti-sense direction, is one deriving from a viral parasite."

16. Cells of a plant host which are resistant to a parasite of said plant host, wherein a gene fragment isolated from said parasite or a DNA or RNA segment substantially homologous to at least part of said gene fragment or to a DNA or RNA sequence functionally equivalent to said gene fragment has been inserted in a manner to provide a heritable characteristic, characterized in that the gene fragment or a DNA or RNA segment substantially homologous to at least part of said gene fragment or to a DNA or RNA sequence functionally equivalent to said gene fragment, which is inserted in a manner to provide a heritable characteristic into said cells of plant host, is transcribed in anti-sense direction and its transcription product interferes with the parasite functions and reproduction.

17. Cells of a plant host of claim 16 whereby the gene fragment or a DNA or RNA segment substantially homologous to at least part of said gene fragment or to a DNA or RNA sequence functionally equivalent to said gene fragment, which is inserted and transcribed in anti-sense direction, is one deriving from a viral parasite".

18. Cells of a bacterial host which are resistant to a parasite of said bacterial host, wherein a gene fragment isolated from said parasite or a DNA or RNA segment substantially homologous to at least part of said gene fragment or to a DNA or RNA sequence functionally equivalent to said gene fragment has been inserted in a manner to provide a heritable characteristic characterized in that the gene fragment or a DNA or RNA segment substantially homologous to at least part of said gene fragment or to a DNA or RNA sequence functionally equivalent to said gene fragment, which is inserted and stably integrated into said cells of bacterial host, is transcribed in anti-sense direction and its transcription product interferes with the parasite functions and reproduction.

19. Cells of a bacterial host of claim 18 whereby the gene fragment or DNA or RNA segment substantially homologous to at least part of said gene fragment or to a DNA or RNA sequence functionally equivalent to said gene fragment, which is inserted and transcribed in anti-sense direction, is one deriving from a viral parasite.

20. A plant made resistant to a parasite of said plant wherein a gene fragment isolated from said parasite or a DNA or RNA segment substantially homologous to at least part of said gene fragment or to a DNA or RNA sequence functionally equivalent to said gene fragment has been inserted in a manner to provide a heritable characteristic into said plant, characterized in that the gene fragment, or DNA or RNA segment substantially homologous to at least part of said gene fragment or to a DNA or RNA sequence functionally equivalent to said gene fragment which is inserted in a manner to provide a heritable characteristic into said plant, is transcribed in anti-sense direction and its transcription product interferes with the parasite functions and reproduction.

21. A plant of claim 20 wherein the gene fragment or a DNA or RNA segment substantially homologous to at least part of said gene fragment or to a DNA or RNA sequence functionally equivalent to said gene fragment, which is inserted and trascribed in anti-sense direction, is one deriving from a viral parasite.

22. A non-human animal made resistant to a parasite of said animal wherein a gene fragment isolated from said parasite or a DNA or RNA segment substantially homologous to at least part of said gene fragment or to a DNA or RNA sequence functionally equivalent to said gene fragment has been inserted in a manner to provide a heritable characteristic into said animal, characterized in that the gene fragment, or DNA or RNA segment substantially homologous to at least part of said gene fragment or to a DNA or RNA sequence functionally equivalent to said gene fragment which is inserted in a manner to provide a heritable characteristic into said animal, is transcribed in anti-sense direction and its transcription product interferes with the parasite functions and reproduction.

23. A non-human animal of claim 22 wherein the gene fragment or a DNA or RNA segment substantially homologous to at least part of said gene fragment or to a DNA or RNA sequence functionally equivalent to said gene fragment, which is inserted and trascribed in anti-sense direction, is one deriving from a viral parasite.

## Patentansprüche

1. Verfahren zum Verleihen einer Resistenz in einem nichtmenschlichen, nichttierischen Wirt gegenüber einem Parasit davon, welches umfaßt:
(a) Isolieren eines Genfragments aus dem Parasit, und
(b) Einfügen des Genfragments oder eines DNA- oder RNA-Segments, welches im wesentlichen zu mindestens einem Teil des Genfragments oder zu einer DNA- oder RNA-Sequenz, die zu dem Genfragment funktionell äquivalenten ist, homolog ist, in den Wirt, auf eine Weise, so daß ein vererbbares charakteristisches Merkmal zur Verfügung gestellt wird,
wobei das Genfragment oder das DNA- oder RNA-Segment in dem Wirt in einer Gegenrichtung transkribiert wird und das Transkriptionsprodukt des Genfragments oder des DNA- oder RNA-Segments die Funktionen und Reproduktion des Parasiten beeinflussen.

2. Verfahren nach Anspruch 1, wobei das Einfügen ein Transformieren des Wirts mit einem Vektor, der das Genfragment trägt, umfaßt, wobei der Vektor in der Lage ist, in dem Wirt stabil zu replizieren, oder das Genfragment in der Lage ist, in ein Chromosom des Wirts eingefügt zu werden.

3. Verfahren nach Anspruch 1 oder 2, wobei der Wirt eine Pflanze oder ein Bakterium ist und der Parasit ein Virus ist.

4. Verfahren nach Anspruch 2, wobei der Wirt ein einzelliger Organismus ist und der Parasit ein Virus ist, der in der Lage ist, den einzelligen Organismus zu infizieren.

5. Verfahren nach Anspruch 2, wobei der Wirt ein Bakterium ist und der Parasit ein Virus ist, der in der Lage ist, das Bakterium zu infizieren.

6. Verfahren nach Anspruch 2, wobei der Vektor ein Plasmid oder Transposon ist, welches in dem Wirt vorkommt, oder ein Teil des Plasmids oder Transposons ist, welches in der Lage ist, als ein Vektor zu funktionieren.

7. Verfahren nach Anspruch 2, wobei der Wirt eine Pflanze ist und der Parasit ein virulenter Virus ist, der in der Lage ist, die Pflanze zu infizieren.

8. Verfahren nach Anspruch 7, wobei der Vektor ein Plasmid, transposables Element oder ein nichtschädliches Virus ist, das in dem Wirt vorkommt, oder Teil des Plasmids, transposablen Elements oder Virus ist, welches in der Lage ist, die Pflanze zu infizieren.

9. Verfahren nach Anspruch 7, wobei der Vektor ein Tiplasmid von Agrobacterium tumefaciens oder ein Teil davon ist, der in der Lage ist, als ein Vektor für die Transformation einer Pflanze zu funktionieren.

10. Verfahren nach Anspruch 1, wobei der Parasit ein virulenter Virus, Pilz oder Bakterium mit Gen-für-Gen Wirt/Parasit-Beziehungen ist und das Gen ein dominantes Avirulenz-Allel von einem avirulenten Stamm des Parasiten ist.

11. Verfahren nach Anspruch 1, wobei das Gen ein Regulatorgen für den Parasit ist oder ein Gen ist, das in die Biosynthese eines Regulatormoleküls in dem Parasiten involviert ist.

12. Verfahren nach Anspruch 1, wobei das Regulatorgen codierend für ein Genprodukt ist, das in die Synthese eines Insektenjuvenil- oder Schmelzhormons, eines Insektenneurotransmitters, eines Regulators zur Haustorialentwicklung oder Sporenbildung bei Pilzen oder eines Pheromons involviert ist.

13. Verfahren nach Anspruch 1, wobei der Wirt Pflanzenzellen umfaßt.

14. Isolierte Zellen eines tierischen Wirts, welche resistent gegenüber einem Parasiten des tierischen Wirts sind, wobei ein von dem Parasit isoliertes Genfragment oder ein DNA- oder RNA-Segment, welches im wesentlichen zu mindestens einem Teil des Genfragments oder zu einer DNA- oder RNA-Sequenz, die zu dem Genfragment funktionell äquivalenten ist, homolog ist, auf eine Weise eingefügt worden ist, so daß ein vererbbares charakteristisches Merkmal zur Verfügung gestellt wird, dadurch gekennzeichnet, daß das Genfragment oder ein DNA- oder RNA-Segment, welches im wesentlichen zu mindestens einem Teil des Genfragments oder zu einer DNA- oder RNA-Sequenz, die zu dem Genfragment funktionell äquivalenten ist, homolog ist, welches auf eine Weise in die Zellen des tierischen Wirts eingefügt ist, um ein vererbbares charakteristisches Merkmal zur Verfügung zu stellen, in einer Gegenrichtung transkribiert wird, und sein Transkriptionsprodukt die Funktionen und die Reproduktion des Parasiten beeinflußt.

15. Isolierte Zellen eines tierischen Wirts nach Anspruch 14, wobei das Genfragment oder ein DNA- oder RNA-Segment, welches im wesentlichen zu mindestens einem Teil des Genfragments oder zu einer DNA- oder RNA-Sequenz, die zu dem Genfragment funktionell äquivalenten ist, homolog ist, welches eingefügt ist und in einer Gegenrichtung transkribiert wird, eines ist, das von einem viralen Parasiten stammt.

16. Zellen eines pflanzlichen Wirts, welche resistent gegenüber einem Parasiten des pflanzlichen Wirts sind, wobei ein von dem Parasit isoliertes Genfragment oder ein DNA- oder RNA-Segment, welches im wesentlichen zu mindestens einem Teil des Genfragments oder zu einer DNA- oder RNA-Sequenz, die zu dem Genfragment funktionell äquivalenten ist, homolog ist, auf eine Weise eingefügt worden ist, so daß ein vererbbares charakteristisches Merkmal zur Verfügung gestellt wird, dadurch gekennzeichnet, daß das Genfragment oder ein DNA- oder RNA-Segment, welches im wesentlichen zu mindestens einem Teil des Genfragments oder zu einer DNA- oder RNA-Sequenz, die zu dem Genfragment funktionell äquivalenten ist, homolog ist, welches auf eine Weise in die Zellen des pflanzlichen Wirts eingefügt ist, um ein vererbbares charakteristisches Merkmal zur Verfügung zu stellen, in einer Gegenrichtung transkribiert wird, und sein Transkriptionsprodukt die Funktionen und die Reproduktion des Parasiten beeinflußt.

17. Zellen eines pflanzlichen Wirts nach Anspruch 16, wobei das Genfragment oder ein DNA- oder RNA-Segment, welches im wesentlichen zu mindestens einem Teil des Genfragments oder zu einer DNA- oder RNA-Sequenz, die zu dem Genfragment funktionell äquivalenten ist, homolog ist, welches eingefügt ist und in einer Gegenrichtung transkribiert wird, eines ist, das von einem viralen Parasiten stammt.

18. Zellen eines bakteriellen Wirts, welche resistent gegenüber einem Parasiten des bakteriellen Wirts sind, wobei ein von dem Parasit isoliertes Genfragment oder ein DNA- oder RNA-Segment, welches im wesentlichen zu mindestens einem Teil des Genfragments oder zu einer DNA- oder RNA-Sequenz, die zu dem Genfragment funktionell äquivalenten ist, homolog ist, auf eine Weise eingefügt worden ist, so daß ein vererbbares charakteristisches Merkmal zur Verfügung gestellt wird, dadurch gekennzeichnet, daß das Genfragment oder ein DNA- oder RNA-Segment, welches im wesentlichen zu mindestens einem Teil des Genfragments oder zu einer DNA- oder RNA-Sequenz, die zu dem Genfragment funktionell äquivalenten ist, homolog ist, welches in die Zellen des bakteriellen Wirts eingefügt und stabil integriert ist, in einer Gegenrichtung transkribiert wird, und sein Transkriptionsprodukt die Funktionen und die Reproduktion des Parasiten beeinflußt.

19. Zellen eines bakteriellen Wirts nach Anspruch 18, wobei das Genfragment oder DNA- oder RNA-Segment, welches im wesentlichen zu mindestens einem Teil des Genfragments oder zu einer DNA- oder RNA-Sequenz, die zu dem Genfragment funktionell äquivalenten ist, homolog ist, welches eingefügt ist und in einer Gegenrichtung transkribiert wird, eines ist, das von einem viralen Parasiten stammt.

20. Pflanze, die gegenüber einem Parasiten der Pflanze resistent gemacht ist, wobei ein von dem Parasiten isoliertes Genfragment oder ein DNA- oder RNA-Segment, welches im wesentlichen zu mindestens einem Teil des Genfragments oder zu einer DNA- oder RNA-Sequenz, die zu dem Genfragment funktionell äquivalenten ist, homolog ist, auf eine Weise eingefügt worden ist, so daß ein vererbbares charakteristisches Merkmal in der Pflanze zur Verfügung gestellt wird, dadurch gekennzeichnet, daß das Genfragment oder DNA-oder RNA-Segment, welches im wesentlichen zu mindestens einem Teil des Genfragments oder zu einer DNA-oder RNA-Sequenz, die zu dem Genfragment funktionell äquivalenten ist, homolog ist, welches auf eine Weise eingefügt ist, um ein vererbbares charakteristisches Merkmal in der Pflanze zur Verfügung zu stellen, in einer Gegenrichtung transkribiert wird, und sein Transkriptionsprodukt die Funktionen und die Reproduktion des Parasiten beeinflußt.

21. Pflanze nach Anspruch 20, wobei das Genfragment oder ein DNA- oder RNA-Segment, welches im wesentlichen zu mindestens einem Teil des Genfragments oder zu einer DNA- oder RNA-Sequenz, die zu dem Genfragment funktionell äquivalenten ist, homolog ist, welches eingefügt ist und in einer Gegenrichtung transkribiert wird, eines ist, das von einem viralen Parasiten stammt.

22. Nichtmenschliches Tier, das gegenüber einem Parasiten des Tieres resistent gemacht ist, wobei ein von dem Parasiten isoliertes Genfragment oder ein DNA- oder RNA-Segment, welches im wesentlichen zu mindestens einem Teil des Genfragments oder zu einer DNA- oder RNA-Sequenz, die zu dem Genfragment funktionell äquivalenten ist, homolog ist, auf eine Weise eingefügt worden ist, so daß ein vererbbares charakteristisches Merkmal in dem Tier zur Verfügung gestellt wird, dadurch gekennzeichnet, daß das Genfragment oder DNA-oder RNA-Segment, welches im wesentlichen zu mindestens einem Teil des Genfragments oder zu einer DNA-oder RNA-Sequenz, die zu dem Genfragment funktionell äquivalenten ist, homolog ist, welches auf eine Weise eingefügt ist, um ein vererbbares charakteristisches Merkmal in dem Tier zur Verfügung zu stellen, in einer Gegenrichtung transkribiert wird, und sein Transkriptionsprodukt die Funktionen und die Reproduktion des Parasiten beeinflußt.

23. Nichtmenschliches Tier nach Anspruch 22, wobei das Genfragment oder ein DNA- oder RNA-Segment, welches im wesentlichen zu mindestens einem Teil des Genfragments oder zu einer DNA- oder RNA-Sequenz, die zu dem Genfragment funktionell äquivalenten ist, homolog ist, welches eingefügt ist und in einer Gegenrichtung transkribiert wird, eines ist, das von einem viralen Parasiten stammt.

## Revendications

1. Procédé pour conférer une résistance vis-à-vis d'un parasite à un hôte non humain et non animal, qui comprend :
a) l'isolation d'un fragment de gène provenant dudit parasite, et
b) l'insertion, d'une manière conférant une caractéristique héréditaire, dudit fragment de gène ou d'un segment d'ADN ou d'ARN pratiquement homologue d'au moins une partie dudit fragment de gène ou d'une séquence d'ADN ou d'ARN fonctionnellement équivalente audit fragment de gène dans ledit hôte,
dans lequel ledit fragment de gène, ou ledit segment d'ADN ou d'ARN dans ledit hôte, est transcrit dans une direction anti-sens et le produit de transcription dudit gène ou dudit segment d'ADN ou d'ARN interfère avec les fonctions et la reproduction du parasite.

2. Procédé selon la revendication 1, dans lequel ladite insertion comprend la transformation de l'hôte avec un vecteur portant ledit fragment de gène, dans lequel ledit vecteur est capable de se répliquer de façon stable dans l'hôte ou ledit fragment de gène est capable d'être inséré dans un chromosome dudit hôte.

3. Procédé selon la revendication 1 ou 2, dans lequel l'hôte est une plante ou une bactérie et le parasite est un virus.

4. Procédé selon la revendication 2, dans lequel ledit hôte est un organisme unicellulaire et ledit parasite est un virus capable d'infecter ledit organisme unicellulaire.

5. Procédé selon la revendication 2, dans lequel ledit hôte est une bactérie et ledit parasite est un virus capable d'infecter ladite bactérie.

6. Procédé selon la revendication 2, dans lequel ledit vecteur est un plasmide ou transposon trouvé dans ledit hôte ou une partie dudit plasmide ou transposon capable de fonctionner comme un vecteur.

7. Procédé selon la revendication 2, dans lequel ledit hôte est une plante et ledit parasite est un virus virulent capable d'infecter ladite plante.

8. Procédé selon la revendication 7, dans lequel ledit vecteur est un plasmide, un élément transposable ou un virus non nocif trouvé dans ledit hôte ou une partie dudit plasmide, dudit élément transposable, ou dudit virus capable d'infecter ladite plante.

9. Procédé selon la revendication 7, dans lequel ledit vecteur est un plasmide Ti de Agrobacterium tumefaciens ou une partie de celui-ci capable de fonctionner comme un vecteur pour la transformation d'une plante.

10. Procédé selon la revendication 1, dans lequel ledit parasite est un virus, champignon ou bactérie virulent(e) ayant des relations hôte/parasite de gène à gène, et ledit gène est un allèle d'avirulence dominant provenant d'une souche avirulente dudit parasite.

11. Procédé selon la revendication 1, dans lequel ledit gène est un gène régulateur pour ledit parasite ou est un gène impliqué dans la biosynthèse d'une molécule régulatrice dans ledit parasite.

12. Procédé selon la revendication 1, dans lequel ledit gène régulateur code un produit génique impliqué dans la synthèse d'une hormone de mue ou de jeunesse des insectes, d'un neurotransmetteur d'insecte, d'un régulateur de sporulation ou de développement haustorial des champignons, ou une phéromone.

13. Procédé selon la revendication 1, dans lequel ledit hôte comprend des cellules végétales.

14. Cellules isolées d'un hôte animal qui sont résistantes à un parasite dudit hôte animal, dans lesquelles un fragment de gène isolé à partir dudit parasite ou un segment d'ADN ou d'ARN pratiquement homologue d'au moins une partie dudit fragment de gène ou d'une séquence d'ADN ou d'ARN fonctionnellement équivalente audit fragment de gène, a été inséré d'une manière conférant une caractéristique héréditaire, caractérisées en ce que le fragment de gène ou un segment d'ADN ou d'ARN pratiquement homologue d'au moins une partie dudit fragment de gène ou d'une séquence d'ADN ou d'ARN fonctionnellement équivalente audit segment de gène, qui est inséré d'une manière conférant une caractéristique héréditaire dans lesdites cellules d'un hôte animal, est transcrit dans une direction anti-sens et son produit de transcription interfère avec la reproduction et les fonctions du parasite.

15. Cellules isolées d'un hôte animal selon la revendication 14, dans lesquelles le fragment de gène ou un segment d'ADN ou d'ARN pratiquement homologue d'au moins une partie dudit fragment de gène ou d'une séquence d'ADN ou d'ARN fonctionnellement équivalente audit fragment de gène, qui est inséré et transcrit dans une direction anti-sens, dérive d'un parasite viral.

16. Cellules d'un hôte végétal qui sont résistantes à un parasite dudit hôte végétal, dans lesquelles un fragment de gène isolé à partir dudit parasite ou un segment d'ADN ou d'ARN pratiquement homologue d'au moins une partie dudit fragment de gène ou d'une séquence d'ADN ou d'ARN fonctionnellement équivalente audit fragment de gène, a été inséré d'une manière conférant une caractéristique héréditaire, caractérisées en ce que le fragment de gène ou un segment d'ADN ou d'ARN pratiquement homologue d'au moins une partie dudit fragment de gène ou d'une séquence d'ADN ou d'ARN fonctionnellement équivalente audit segment de gène, qui est inséré d'une manière conférant une caractéristique héréditaire dans lesdites cellules d'un hôte végétal, est transcrit dans une direction anti-sens et son produit de transcription interfère avec la reproduction et les fonctions du parasite.

17. Cellules d'un hôte végétal selon la revendication 16, dans lesquelles le fragment de gène ou un segment d'ADN ou d'ARN pratiquement homologue d'au moins une partie dudit fragment de gène ou d'une séquence d'ADN ou d'ARN fonctionnellement équivalente audit fragment de gène, qui est inséré et transcrit dans une direction anti-sens, dérive d'un parasite viral.

18. Cellules d'un hôte bactérien qui sont résistantes à un parasite dudit hôte bactérien, dans lesquelles un fragment de gène isolé à partir dudit parasite ou un segment d'ADN ou d'ARN pratiquement homologue d'au moins une partie dudit fragment de gène ou d'une séquence d'ADN ou d'ARN fonctionnellement équivalente audit fragment de gène, a été inséré d'une manière conférant une caractéristique héréditaire, caractérisées en ce que le fragment de gène ou un segment d'ADN ou d'ARN pratiquement homologue d'au moins une partie dudit fragment de gène ou d'une séquence d'ADN ou d'ARN fonctionnellement équivalente audit segment de gène, qui est inséré et intégré de façon stable dans lesdites cellules d'un hôte bactérien, est transcrit dans une direction anti-sens et son produit de transcription interfère avec la reproduction et les fonctions du parasite.

19. Cellules d'un hôte bactérien selon la revendication 18, dans lesquelles le fragment de gène ou un segment d'ADN ou d'ARN pratiquement homologue d'au moins une partie dudit fragment de gène ou d'une séquence d'ADN ou d'ARN fonctionnellement équivalente audit fragment de gène, qui est inséré et transcrit dans une direction anti-sens, dérive d'un parasite viral.

20. Plante qui est rendue résistante à un parasite de ladite plante, dans laquelle un fragment de gène isolé à partir dudit parasite ou un segment d'ADN ou d'ARN pratiquement homologue d'au moins une partie dudit fragment de gène ou d'une séquence d'ADN ou d'ARN fonctionnellement équivalente audit fragment de gène a été inséré d'une manière conférant une caractéristique héréditaire dans ladite plante, caractérisée en ce que le fragment de gène, ou le segment d'ADN ou d'ARN pratiquement homologue d'au moins une partie dudit fragment de gène ou d'une séquence d'ADN ou d'ARN fonctionnellement équivalente audit fragment de gène qui est inséré de manière à conférer une caractéristique héréditaire dans ladite plante, est transcrit dans une direction anti-sens et son produit de transcription interfère avec la reproduction et les fonctions du parasite.

21. Plante selon la revendication 20, dans laquelle le fragment de gène ou un segment d'ADN ou d'ARN pratiquement homologue d'au moins une partie dudit fragment de gène ou d'une séquence d'ADN ou d'ARN fonctionnellement équivalente audit fragment de gène, qui est inséré et transcrit dans une direction anti-sens, dérive d'un parasite viral.

22. Animal non humain qui est rendu résistant à un parasite dudit animal, dans lequel un fragment de gène isolé à partir dudit parasite ou un segment d'ADN ou d'ARN pratiquement homologue d'au moins une partie dudit fragment de gène ou d'une séquence d'ADN ou d'ARN fonctionnellement équivalente audit fragment de gène a été inséré d'une manière conférant une caractéristique héréditaire dans ledit animal, caractérisé en ce que le fragment de gène, ou le segment d'ADN ou d'ARN pratiquement homologue d'au moins une partie dudit fragment de gène ou d'une séquence d'ADN ou d'ARN fonctionnellement équivalente audit fragment de gène qui est inséré de manière à conférer une caractéristique héréditaire dans ledit animal, est transcrit dans une direction anti-sens et son produit de transcription interfère avec la reproduction et les fonctions du parasite.

23. Animal non humain selon la revendication 22, dans laquelle le fragment de gène ou un segment d'ADN ou d'ARN pratiquement homologue d'au moins une partie dudit fragment de gène ou d'une séquence d'ADN ou d'ARN fonctionnellement équivalente audit fragment de gène, qui est inséré et transcrit dans une direction anti-sens, dérive d'un parasite viral.
